# EUROPÄISCHE PATENTSCHRIFT

(11) **EP 3 773 799 B1**
(45) Veröffentlichungstag und Bekanntmachung des Hinweises auf die Patenterteilung: **28.12.2022**
(21) Anmeldenummer: 19718589.5
(22) Anmeldetag: 04.04.2019
(51) Int. Cl.: A61M 5/145, A61M 5/142

(54) **VERFAHREN ZUM KALIBRIEREN EINER SPRITZENPUMPE, SPRITZENPUMPE UND VORRICHTUNGEN**
METHOD FOR CALIBRATING A SYRINGE PUMP, SYRINGE PUMP AND DEVICE
PROCÉDÉ POUR CALIBRER UNE POMPE SERINGUE, POMPE SERINGUE ET DISPOSITIF

(30) Priorität: 06.04.2018 DE 102018108203
(43) Veröffentlichungstag der Anmeldung: 17.02.2021
(73) Patentinhaber: Fresenius Medical Care Deutschland GmbH, 61352 Bad Homburg (DE)
(72) Erfinder: TANNEBERG, Michael, 07745 Jena (DE); SIEBERT, Jochen, 97688 Bad Kissingen (DE); ROGMANN, Ralf, 97525 Schwebheim (DE); DIETZ, Patrick, 97711 Weichtungen (DE); RUECKERT, Jochen, 97520 Roethlein (DE)
(74) Vertreter: Bobbert & Partner Patentanwälte PartmbB
(86) Internationale Anmeldenummer: PCT/EP2019/058488
(87) Internationale Veröffentlichungsnummer: WO 2019/193089

(56) Entgegenhaltungen:
- EP-A1- 3 202 440
- US-A- 5 034 004
- US-A1- 2014 197 824

## Beschreibung

Die vorliegende Erfindung betrifft ein Verfahren zum Kalibrieren einer medizinischen Spritzenpumpe gemäß Anspruch 1. Sie betrifft zudem eine medizinische Spritzenpumpe gemäß Anspruch 8 sowie eine Blutbehandlungsvorrichtung gemäß Anspruch 16. Des Weiteren betrifft die vorliegende Erfindung ein digitales Speichermedium gemäß Anspruch 18 und ein Computerprogramm-Produkt gemäß Anspruch 19.

Aus der Praxis sind Spritzenpumpen bekannt. Die zugehörige Pumpensteuerung benötigt Signale, welche auf definierte Positionen der Linearbewegung des verfahrbaren Schlittens der Spritzenpumpe, mittels welchem die Spritze entleert wird, hinweisen. Diese Signale werden mittels Sensoren registriert, welche z. B. beispielsweise Magnetfeldsensoren (z. B. Hall-Sensoren) oder optoelektronische Sensoren (z. B. Lichtschranken) sein können. Die Sensoren dieser Spritzenpumpen dienen der Pumpensteuerung somit zum Ermitteln der aktuellen Position des Schlittens und hierüber dem korrekten Ansteuern des Pumpenmotors. Beispielhafte Spritzenpumpen sind aus der US 2014/197824 A1 und der EP 3202440 A1 bekannt. Erstere zeigt dabei die Kombination der Merkmale des Oberbegriffs von Anspruch 1.

Zur Gewährleistung einer korrekten und präzisen Ansteuerung des Motors muss die Spritzenpumpe vorab einem bislang aufwendigen Kalibrierprozess unterzogen werden.

Eine Aufgabe der vorliegenden Erfindung ist es, ein weiteres, vorzugsweise automatisiertes oder weitestgehend automatisiertes, Verfahren zum Kalibrieren einer medizinischen Spritzenpumpe vorzuschlagen. Zudem sollen eine medizinische Spritzenpumpe, die konfiguriert und/oder vorbereitet ist, ein solches Verfahren auszuführen oder mittels eines solchen Verfahrens kalibriert ist, und eine Blutbehandlungsvorrichtung angegeben werden. Des Weiteren sollen ein geeignetes digitales Speichermedium, ein geeignetes Computerprogramm-Produkt und ein geeignetes Computerprogramm zum Ausführen des Verfahrens angegeben werden.

Die erfindungsgemäße Aufgabe wird durch ein Verfahren mit den Merkmalen des Anspruchs 1 gelöst. Sie wird ferner gelöst durch eine medizinische Spritzenpumpe mit den Merkmalen des Anspruchs 8 und durch eine Blutbehandlungsvorrichtung mit den Merkmalen des Anspruchs 16. Des Weiteren wird die erfindungsgemäße Aufgabe durch ein digitales Speichermedium mit den Merkmalen des Anspruchs 18und durch ein Computerprogramm-Produkt mit den Merkmalen des Anspruchs 19 gelöst.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert" oder "konfiguriert" die Rede ist, so ist auch offenbart, diese Begriffe gegeneinander auszutauschen.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Das erfindungsgemäße Verfahren zum Kalibrieren einer medizinischen Spritzenpumpe betrifft eine Spritzenpumpe mit einer Spritzenaufnahme zum Aufnehmen einer Spritze, welche einen Spritzenzylinder und einen hierin zwischen einer ersten Endlage und einer zweiten Endlage verfahrbaren Kolben aufweist, wobei der Kolben mit einer Kolbenstange verbunden ist oder eine Kolbenstange aufweist.

Ferner umfasst die zu kalibrierende medizinische Spritzenpumpe einen Spritzenmotor und einen mittels des Spritzenmotors in, insbesondere voneinander unterscheidbaren bzw. gegeneinander abgrenzbaren, Motorschritten verfahrbaren Schlitten. Der Schlitten weist einen Knebel zum Aufnehmen eines Abschnitts der Kolbenstange der Spritze oder zum Verbinden des Knebels hiermit auf. Dieser Abschnitt kann eine Druckplatte sein, die mit der Kolbenstange verbunden ist Ein Abschnitt des Schlittens ist, z. B. relativ zur Spritzenaufnahme, mittels des Schlittens maximal zwischen einem ersten Endanschlag und einem zweiten Endanschlag verfahrbar angeordnet. Der Schlitten ist konfiguriert, um in Motorschritten (diese können Mikroschritte sein) verfahren zu werden. Dies geschieht längs zur Lage einer im Gebrauch der Spritzenpumpe in diese eingelegten Spritze.

Des Weiteren umfasst die zu kalibrierende Spritzenpumpe eine Zählvorrichtung zum Zählen oder Mitzählen der Anzahl von Motorschritten, um welche der Schlitten verfahren wird, beispielsweise ähnlich einem Kilometerzähler. Die Zählvorrichtung kann vorzugsweise vor- und zurückzählen. Sie kann weiter vorzugsweise auf Null oder einen anderen Startwert gestellt werden.

Außerdem umfasst die zu kalibrierende Spritzenpumpe einen ersten Sensor und einen zweiten Sensor, welche beide entlang eines Verschiebewegs des Kolbens der Spritze oder eines Verfahrwegs des Schlittens der Pumpe angeordnet sind. Sie sind optional dazu konfiguriert und/oder angeordnet, um mit dem Schlitten oder einem Abschnitt hiervon in einer Sensorbeziehung zu stehen.

Die zu kalibrierende Spritzenpumpe umfasst zudem eine Steuervorrichtung, welche konfiguriert ist, um den Schlitten zu verfahren, indem sie Steueranweisungen folgt. Diese Steueranweisungen können beispielsweise vom Anwender mittels einer Eingabevorrichtung eingegeben und/oder von einer Speichervorrichtung vorgehalten und aus dieser ausgelesen werden.

Hierzu, und gegebenenfalls zum Speichern der von den Sensoren erfassten Daten oder der errechneten Kalibrierwerte, umfasst die Spritzenpumpe eine Speichervorrichtung.

Schließlich ist eine Eingabevorrichtung zum Eingeben von Steueranweisungen und/oder von anderen Daten durch einen Anwender ebenfalls vorgesehen.

Das erfindungsgemäße Verfahren zum Kalibrieren der medizinischen Spritzenpumpe umfasst folgende Schritte:
a) Das optionale Eingeben wenigstens einer Information, welche einen konkreten Typ der Spritze betrifft. Die Eingabe kann mittels der Eingabevorrichtung erfolgen. Sie kann mittels der Speichervorrichtung gespeichert werden.
b) Verfahren eines Abschnitts des Schlittens in eine Start- oder Betriebsposition, beispielsweise eine mittlere Position zwischen der ersten Endlage und der zweiten Endlage, insbesondere in eine Position zwischen dem ersten und dem zweiten Sensor, jedenfalls sofern diese Position nicht schon erreicht ist. Dies geschieht insbesondere mit einer ersten Geschwindigkeit.
c) Verfahren des Knebels mittels des Schlittens derart, dass der Kolben bei eingelegter Spritze in die erste Endlage gelangen würde oder bis ein vorbestimmter Abschnitt des Schlittens, beispielsweise der Permanentmagnet, an den ersten Endanschlag gelangt ist. Dies geschieht insbesondere mit einer zweiten Geschwindigkeit. Wenn hierbei ein vorbestimmter Abschnitt des Schlittens, z. B. ein Permanentmagnet, die Position des zweiten Sensors erreicht oder passiert, wird diese Position als erster Zählwert ermittelt oder diesem gleichgesetzt. Dies ist beispielsweise genau dann der Fall, wenn der Permanentmagnet am, exemplarisch als Hall-Sensor ausgestalteten, zweiten Sensor vorbeifährt (oder dessen Höhe erreicht) oder wenn eine Lichtschranke, die dem zweiten Sensor entspricht, vom vorbestimmten Abschnitt unterbrochen wird. Dieser erste Zählwert kann z. B. eine von der Zählvorrichtung gelieferte Anzahl an Motorschritten sein. Gegebenenfalls wird der erste Zählwert, optional vorübergehend, gespeichert. Weiter werden bei Erreichen der ersten Endlage durch den Kolben oder des vorbestimmten Abschnitts, oder bei Erreichen des ersten Endanschlags durch den Abschnitt des Schlittens, die bis zum Erreichen der Position der ersten Endlage oder des ersten Endanschlags mittels der Zählvorrichtung seit Verlassen der Position des zweiten Sensors gezählten Motorschritte als ein zweiter Zählwert ermittelt. Auch dieser wird optional, beispielweise vorübergehend, gespeichert.
d) Errechnen eines Kalibrierwerts des zweiten Sensors aus dem ersten Zählwert und dem zweiten Zählwert, z. B. als Differenz zwischen oder Summe von erstem Zählwert und zweitem Zählwert, oder als Betrag hiervon, beispielsweise durch eine hierfür programmierte Rechnereinheit, oder durch die hierzu optional konfigurierte Zählvorrichtung, und Speichern dieses Kalibrierwerts für den zweiten Sensor mittels der Speichervorrichtung.
e) Optional wird die Zählvorrichtung, z. B. auf "0" oder einen anderen Startwert, zurückgesetzt.
f) Verfahren des Knebels oder des Schlittens, insbesondere mit der zweiten oder einer dritten Geschwindigkeit, in Richtung der zweiten Endlage.
g) Ermitteln der Anzahl an Motorschritten welche die Zählvorrichtung bei Erreichen der Position des ersten Sensors oder bei dessen Passieren (beispielsweise ein Vorbeifahren des Permanentmagneten an einem Hall-Sensor, oder Durchfahren einer Lichtschranke) mittels des vorbestimmten Abschnitts des Schlittens ausgibt, als dritten Zählwert.
h) Errechnen eines Kalibrierwerts des ersten Sensors aus dem dritten Zählwert und optional wenigstens einem weiteren Wert aus der Gruppe, die aus dem ersten Zählwert, dem zweiten Zählwert und dem Kalibrierwert des zweiten Sensors besteht, z. B. als die Differenz zwischen zweitem und drittem Zählwert oder als der Betrag des dritten Zählwerts, beispielsweise durch eine hierfür geeignete Rechnereinheit oder durch die Zählvorrichtung, und Speichern dieses Kalibrierwerts für den ersten Sensor mittels der Speichervorrichtung.

Das Errechnen der Kalibrierwerte kann im einfachsten Fall ein einfaches Ablesen des Zählwerts sein, beispielweise wenn der jeweilige Startwert "0" betrug.

Die erste Geschwindigkeit kann zwischen 100 µs/Mikroschritt und 1000 µs/Mikroschritt, vorzugsweise 500 µs/Mikroschritt, betragen. Die zweite Geschwindigkeit kann zwischen 1000 µs/Mikroschritt und 10000 µs/Mikroschritt, vorzugsweise 5000 µs/Mikroschritt, betragen. Alternativ kann die zweite Geschwindigkeit zwischen 1000 µs/Mikroschritt und 2000 µs/Mikroschritt, insbesondere 1400 µs/Mikroschritt, betragen. Die dritte Geschwindigkeit kann zwischen 10000 µs/Mikroschritt und 50000 µs/Mikroschritt, vorzugsweise 20000 µs/Mikroschritt, betragen.

Die erfindungsgemäße medizinische Spritzenpumpe umfasst eine Spritzenaufnahme zum Aufnehmen einer Spritze, welche einen Spritzenzylinder und einen hierin zwischen einer ersten Endlage und einer zweiten Endlage verfahrbaren Kolben aufweist, wobei der Kolben mit einer Kolbenstange verbunden ist.

Ferner umfasst die erfindungsgemäße medizinische Spritzenpumpe einen Spritzenmotor und einen mittels des Spritzenmotors in, insbesondere unterscheidbaren bzw. gegeneinander abgrenzbaren, Motorschritten verfahrbaren Schlitten. Der Schlitten weist einen Knebel zum Aufnehmen eines Abschnitts der Kolbenstange der Spritze oder zum Verbinden des Knebels hiermit auf. Dieser Abschnitt kann eine Druckplatte sein, die mit der Kolbenstange verbunden ist. Der Knebel ist relativ zur Spritzenaufnahme mittels des Schlittens maximal zwischen einem ersten Endanschlag und einem zweiten Endanschlag verfahrbar angeordnet. Der Schlitten ist konfiguriert, um in Motorschritten (können Mikroschritte sein) verfahren zu werden. Dies geschieht längs zur Lage einer im Gebrauch der Spritzenpumpe in diese eingelegten Spritze.

Des Weiteren umfasst die erfindungsgemäße medizinische Spritzenpumpe eine Zählvorrichtung zum Zählen oder Mitzählen der Anzahl von Motorschritten, um welche der Schlitten verfahren wird, beispielsweise ähnlich einem Kilometerzähler, der vorzugsweise vor- und zurückzählen kann und der weiter vorzugsweise auf Null oder einen anderen Startwert gestellt werden kann.

Außerdem umfasst die erfindungsgemäße medizinische Spritzenpumpe einen ersten Sensor und einen zweiten Sensor, welche beide entlang eines Verschiebewegs des Kolbens der Spritze oder eines Verfahrwegs des Schlittens der Pumpe angeordnet sind und welche optional dazu konfiguriert und/oder angeordnet sind, um mit dem Schlitten oder einem Abschnitt hiervon in einer Sensorbeziehung zu stehen.

Die Spritzenpumpe umfasst zudem eine Steuervorrichtung, welche zum Verfahren des Schlittens konfiguriert ist, indem sie Steueranweisungen folgt. Diese Steueranweisungen können beispielsweise vom Anwender mittels einer Eingabevorrichtung eingegeben und/oder von einer Speichervorrichtung vorgehalten und aus dieser ausgelesen werden.

Hierzu, und gegebenenfalls zum Speichern der von den Sensoren erfassten Daten oder der errechneten Kalibrierwerte, umfasst die Spritzenpumpe eine Speichervorrichtung.

Schließlich ist eine Eingabevorrichtung zum Eingeben von Steueranweisungen und/oder von anderen Daten durch einen Anwender ebenfalls vorgesehen.

Die Steuervorrichtung der erfindungsgemäßen medizinischen Spritzenpumpe ist konfiguriert und/oder vorbereitet, um das erfindungsgemäße Verfahren auszuführen, oder um ein Verfahren zum Kalibrieren der medizinischen Spritzenpumpe auszuführen, welches wiederum folgende Schritte umfasst:
a) Das optionale Eingeben wenigstens einer Information, welche einen konkreten Typ der Spritze betrifft. Die Eingabe kann mittels der Eingabevorrichtung erfolgen. Die Eingabe kann z. B. mittels einer Auswahlliste für den Anwender, etwa in Gestalt eines Menüfeldes, eines Touchscreenfeldes (beispielsweise der Spritzenpumpe oder einer hiermit in Signalkommunikation verbundenen, z. B. erfindungsgemäßen, Blutbehandlungsvorrichtung) usw. erfolgen. Die Auswahl kann z. B. über das Servicemenü erfolgen. Der Servicetechniker muss hierzu vorteilhafterweise nicht vor Ort sein. Der Eingabe kann ein Speichern dieser Information mittels der Speichervorrichtung folgen.
b) Verfahren eines Abschnitts des Schlittens in eine, beispielsweise mittlere, Position zwischen der ersten Endlage und der zweiten Endlage, insbesondere in eine Position zwischen dem ersten und dem zweiten Sensor, jedenfalls sofern diese Position nicht schon erreicht ist. Dies geschieht insbesondere mit einer ersten Geschwindigkeit.
c) Verfahren des Knebels mittels des Schlittens derart, dass der Kolben bei eingelegter Spritze in die erste Endlage gelangen würde oder bis ein vorbestimmter Abschnitt des Schlittens, beispielsweise der Permanentmagnet, an den ersten Endanschlag gelangt ist. Dies geschieht insbesondere mit einer zweiten Geschwindigkeit. Wenn hierbei ein vorbestimmter Abschnitt des Schlittens, z. B. ein Permanentmagnet, die Position des zweiten Sensors erreicht oder passiert, wird diese Position als erster Zählwert ermittelt oder diesem gleichgesetzt. Dies ist beispielsweise genau dann der Fall, wenn der Permanentmagnet am, exemplarisch als Hall-Sensor ausgestalteten, zweiten Sensor vorbeifährt oder wenn eine Lichtschranke, die dem zweiten Sensor entspricht, vom vorbestimmten Abschnitt unterbrochen wird. Dieser erste Zählwert kann z. B. eine von der Zählvorrichtung gelieferte Anzahl an Motorschritten sein. Gegebenenfalls wird der erste Zählwert, optional vorübergehend, gespeichert. Weiter werden bei Erreichen der ersten Endlage durch den Kolben oder bei Erreichen des ersten Endanschlags durch den Abschnitt des Schlittens die bis zum Erreichen der Position der ersten Endlage oder des ersten Endanschlags mittels der Zählvorrichtung gezählten Motorschritte als ein zweiter Zählwert ermittelt. Auch dieser wird optional, beispielweise vorübergehend, gespeichert.
d) Errechnen eines Kalibrierwerts des zweiten Sensors aus dem ersten Zählwert und dem zweiten Zählwert, z. B. aus Differenz zwischen oder Summe aus erstem Zählwert und zweitem Zählwert oder als Betrag hiervon, beispielsweise durch eine hierfür geeignete Rechnereinheit, oder durch die Zählvorrichtung, und Speichern dieses Kalibrierwerts für den zweiten Sensor mittels der Speichervorrichtung.
e) Optional wird die Zählvorrichtung, z. B. auf "0" oder einen anderen Startwert, zurückgesetzt.
f) Verfahren des Knebels oder des Schlittens, insbesondere mit der zweiten oder mit einer dritten Geschwindigkeit, in Richtung der zweiten Endlage.
g) Ermitteln der Anzahl an Motorschritten welche die Zählvorrichtung bei Erreichen der Position des ersten Sensors oder bei dessen Passieren (beispielsweise ein Vorbeifahren des Permanentmagneten an einem Hall-Sensor, oder Durchfahren einer Lichtschranke) mittels des vorbestimmten Abschnitts des Schlittens ausgibt, als dritten Zählwert.
h) Errechnen eines Kalibrierwerts des ersten Sensors aus dem dritten Zählwert und optional wenigstens einem weiteren Wert aus der Gruppe, die aus dem ersten Zählwert, dem zweiten Zählwert und dem Kalibrierwert des zweiten Sensors besteht, z. B. als die Differenz zwischen zweitem und drittem Zählwert oder als der Betrag des dritten Zählwerts, beispielsweise durch eine hierfür geeignete Rechnereinheit oder durch die Zählvorrichtung, und Speichern dieses Kalibrierwerts für den ersten Sensor mittels der Speichervorrichtung.

Die erfindungsgemäße Blutbehandlungsvorrichtung weist eine erfindungsgemäße medizinische Spritzenpumpe und/oder eine Spritzenpumpe, die mittels des erfindungsgemäßen Verfahrens kalibriert wurde, auf und/oder ist mit einer solchen in Signalkommunikation verbunden.

Ein erfindungsgemäßes digitales, insbesondere nichtflüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode oder eine Signalwelle zur Veranlassung der maschinellen Schritte des erfindungsgemäßen Verfahrens auf, wenn das Computerprogramm-Produkt auf einem Rechner abläuft. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Server-system, Cloud Computing System, etc.) oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm weist einen Programmcode zur Veranlassung der maschinellen Schritte des erfindungsgemäßen Verfahrens auf, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das erfindungsgemäße digitale Speichermedium, das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden. Dies gilt insbesondere im Zusammenwirken mit einer Erfassungsvorrichtung und/oder einer erfindungsgemäßen Blutbehandlungsvorrichtung wie hierin beschrieben.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Wann immer hierin von einer Ausführungsform die Rede ist, so handelt es sich um eine beispielhafte, erfindungsgemäße Ausführungsform.

In einigen Ausführungsformen wird zum Verfahren gemäß dem Schritt b) der Abschnitt des Schlittens zunächst in Richtung des ersten Endanschlags verfahren, und anschließend, insbesondere mit einer zweiten Geschwindigkeit fortgesetzt, nachdem entweder der Abschnitt oder der vorbestimmte Abschnitt, z. B. der Permanentmagnet, entweder die Position des zweiten Sensors oder den ersten Endanschlag erreicht hat. Dabei wird der Abschnitt des Schlittens nach dem Erreichen des zweiten Sensors oder nach Erreichen des ersten Endanschlags automatisch um eine vorbestimmte Anzahl an Motorschritten (oder Mikroschritten) in Richtung des zweiten Endanschlags verfahren, bevor er anschließend wie in Schritt c) weiter verfahren wird.

In manchen Ausführungsformen erreicht der Knebel oder der Abschnitt seine maximalen Positionen jeweils, wenn der Schlitten den ersten Endanschlag bzw. den zweiten Endanschlag erreicht.

Das Erkennen, dass der erste oder der zweite Endanschlag erreicht ist, kann mittels einer gelochten Schlitzscheibe oder Lochscheibe erfolgen, die optional Teil der erfindungsgemäßen oder der zu kalibrierenden Spritzenpumpe ist. Die Schlitzscheibe kann, z. B. über eine Kupplung, von der Spindel der Spritzenpumpe, welche in Verbindung mit dem Pumpenmotor auch den Schlitten in Motorschritten antreibt, angetrieben werden. Die Schlitzscheibe unterbricht, wenn sie sich dreht, intermittierend ein Lichtschrankensignal, z. B. nach einem vorbekannten Muster. Bleibt die Schlitzscheibe stehen, da die Spindel, welche die Schlitzscheibe antreibt, bei Erreichen ihrer mechanischen Endlage ihrerseits stehenbleibt, so bleibt das Lichtschrankensignal statisch oder weicht auf andere Weise vom vorbekannten oder für einen Bewegungszustand des Schlittens erwarteten Muster ab. Die Steuervorrichtung der Spritzenpumpe kann hieran den Stillstand der Schlitzscheibe und hieran wiederum, den Stillstand der Spindel und folglich des Schlittens erkennen. Einer der Endanschläge ist somit angefahren, d. h. erreicht.

In manchen Ausführungsformen ist die Spritze eine Einwegspritze.

Anstelle einer Lichtschranke oder eines anderen optischen Sensors kann die Spindelbewegung und ihr Stillstand mittels anders arbeitenden Sensoren, etwa Magnetfeldsensoren, wie Hall-Sensoren, ermittelt werden. Die o. g. Schlitzscheibe kann dabei z. B. durch eine abwechselnd magnetisierte Index-Scheibe ersetzt werden.

Anstelle von optischen Sensoren und/oder Magnetfeldsensoren kann beispielsweise wenigstens eine Widerstandspiste verwendet werden, die nach dem Prinzip eines Potentiometers mit Schleife und Widerstandspiste arbeitet.

In einigen Ausführungsformen ist die erste Geschwindigkeit höher als die zweite Geschwindigkeit und/oder höher als die dritte Geschwindigkeit.

In manchen Ausführungsformen ist die dritte Geschwindigkeit höher als die zweite Geschwindigkeit. In anderen ist sie so hoch wie die zweite Geschwindigkeit.

In einigen Ausführungsformen wird wenigstens eine ermittelte Anzahl an Motorschritten und/oder wenigstens ein Zählwert mit einem Kompensationswert verrechnet, um auf diese Weise korrigiert oder kompensiert in die Berechnung der Kalibrierwerte des ersten Sensors und/oder des zweiten Sensors gemäß der Schritte d) und g) einzugehen.

Eine beispielhafte Kompensierung kann wie folgt aussehen: Das Erreichen des Endanschlags wird beispielsweise anhand des Ausbleibens *("time out")* des für eine anhaltende Bewegung des Schlittens zu erwartenden Schlitzscheibensignals oder anhand dessen Änderung erkannt.

Das *time out* ist offensichtlich von der Geschwindigkeit abhängig, mit welcher der Schlitten beim Kalibrieren verfahren wird: Wird er schnell verfahren, etwa mit der ersten Geschwindigkeit so kann von einem Stillstand des Schlittens ausgegangen werden bei einem *time out,* das beispielsweise über mindestens 750 ms anhält. Wird er mit der zweiten Geschwindigkeit verfahren, so kann von einem Stillstand des Schlittens ausgegangen werden bei einem *time out,* das beispielsweise über mindestens 1500 ms anhält. Wird er langsam verfahren, etwa mit der dritten Geschwindigkeit, so kann von einem Stillstand des Schlittens ausgegangen werden bei einem *time out,* das beispielsweise über mindestens 3750 ms anhält.

Das Erreichen des Endanschlags kann durch das *time out* also erst verzögert sicher erkannt werden. Das führt zu einer Abweichung in der Positionserkennung, die je nach Geschwindigkeit unterschiedlich groß ist.

Um den dadurch entstehenden Fehler zu beseitigen, wurde experimentell ein Kompensationswert ermittelt, der abhängig von der Geschwindigkeit sein kann. Die ermittelten Zählwerte, Sensorpositionen oder Kalibrierwerte können um derartige Kompensationswerte korrigiert werden.

In manchen Ausführungsformen werden die in obiger Weise korrigierten oder kompensierten Kalibrierwerte des ersten Sensors und/oder des zweiten Sensors auf Plausibilität überprüft.

In einigen Ausführungsformen ist der erste und/oder der zweite Sensor ein Hall-Sensor oder ein anderer Magnetfeldsensor, oder eine Lichtschranke oder ein anderer optoelektronischer Sensor. Anstelle von optischen Sensoren und/oder Magnetfeldsensoren kann beispielsweise wenigstens eine Widerstandspiste verwendet werden, die nach dem Prinzip eines Potentiometers mit Schleife und Widerstandspiste arbeitet.

In manchen Ausführungsformen weist der Schlitten der Spritzenpumpe einen Permanentmagnet auf. Dieser steht in einem unveränderlichen Abstand zum Knebel oder zum o. g. Abschnitt des Schlittens.

In gewissen Ausführungsformen umfasst die Information über den konkreten Spritzentyp wenigstens den Abstand zwischen erster und zweiter Endlage der Spritze.

In manchen Ausführungsformen ist die medizinische Spritzenpumpe als Heparinspritzenpumpe ausgestaltet.

In gewissen Ausführungsformen ist die Blutbehandlungsvorrichtung als Peritonealdialysevorrichtung, Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = *continuous renal replacement therapy).*

In einigen Ausführungsformen ist der vorbestimmte Abschnitt die Position des Permanentmagneten auf dem Schlitten.

In manchen Ausführungsformen werden die Sensoren, insbesondere als Hall-Sensoren, während oder zu der Kalibrierung nicht von der Steuervorrichtung getrennt und/oder ihre Leitungsverbindung nicht getrennt.

In einigen Ausführungsformen werden die Positionen der Sensoren, insbesondere als Hall-Sensoren, während oder zu der Kalibrierung nicht verändert. Ihre relative Lage zu anderen Abschnitten der Spritzenpumpe, etwa ihrem Gehäuse, bleibt also erhalten.

In manchen Ausführungsformen ist die Spritze während oder zu der Kalibrierung in die Spritzenaufnahme aufgenommen.

In einigen Ausführungsformen umfasst die medizinische Spritzenpumpe keine Drucksensoren.

In manchen Ausführungsformen umfassen die Steuervorrichtung und/oder die Spritzenpumpe kein Druckventil und sind auch nicht mit einem solchen, insbesondere elektrisch, verbunden.

In einigen Ausführungsformen umfassen die Steuervorrichtung und/oder die Spritzenpumpe keine Druckerkennungseinheit und/oder -erfassungseinheit und sind auch nicht mit einer solchen, insbesondere elektrisch, verbunden.

In manchen Ausführungsformen umfasst das erfindungsgemäße Verfahren keinen Druckprüfmechanismus.

In einigen Ausführungsformen werden in dem erfindungsgemäßen Verfahren keine Druck- und/oder elektrischen Spannungsdaten erfasst, ermittelt, verglichen oder auf eine andere Art und Weise verarbeitet.

Manche oder alle erfindungsgemäßen Ausführungsformen können einen, mehrere oder alle der oben und/oder im Folgenden genannten Vorteile aufweisen.

Mit Hilfe des erfindungsgemäßen Verfahrens lassen sich die beiden Sensoren der Spritzenpumpe auf einfache und schnelle Weise kalibrieren. Dies spart Zeit und Arbeitsaufwand.

Durch das Verfahren des Schlittens während des Kalibrierens mit unterschiedlichen Geschwindigkeiten kann ferner Zeit dadurch eingespart werden, dass der Schlitten einerseits so rasch wie möglich bewegt wird, was die Kalibrierung verkürzen kann. Gleichzeitig wird in anderen Momenten der Kalibrierung so langsam wie nötig verfahren, um die erforderliche Präzision zu gewährleisten, etwa beim Anfahren von für die Kalibrierung kritischen Positionen. Dieser Wechsel zwischen verschiedenen Geschwindigkeiten des Schlittens beim Kalibrieren stellt somit einen weiteren Vorteil dar.

Vorteilhafterweise werden montagebedingte Abweichungen in den Positionen der Sensoren sowie mechanische Toleranzen, beispielsweise die Sensorsignalbreite, bei der erfindungsgemäßen Kalibrierung berücksichtigt. Das automatische Kalibrieren erspart eine aufwändige Justierung von Hand.

Eine besondere Präzision beim werkseitigen Montieren der Sensoren ist erfindungsgemäß entbehrlich. Montagespielräume sind zulässig und können durch das erfindungsgemäße Verfahren an Bedeutung verlieren.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt eine herkömmliche Spritze;
- Fig. 2: zeigt eine Ausführungsform der erfindungsgemäßen Spritzenpumpe in einer schematisch vereinfachten Draufsicht;
- Fig. 3: zeigt den Ablauf einer exemplarischen Ausführungsform des erfindungsgemäßen Verfahrens in einem Flussdiagramm; und
- Fig. 4: zeigt den Ablauf einer exemplarischen Ausführungsform des erfindungsgemäßen Verfahrens in einer Übersicht.

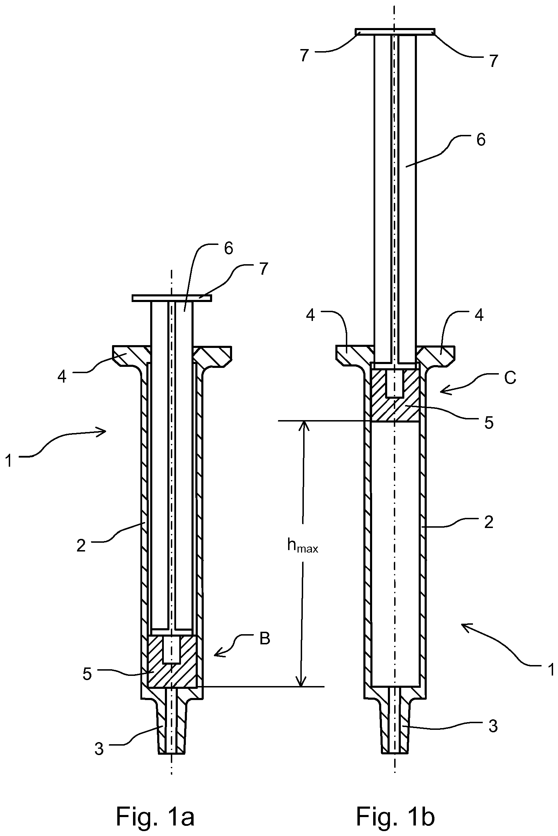

**Fig. 1**a und **Fig. 1b** zeigen, jeweils von der Seite, eine herkömmliche, z. B. mit Heparin befüllbare, Spritze 1, die eine Einmalspritze und/oder eine Fertigspritze sein kann. Fig. 1a zeigt sie in einem leeren Zustand, Fig. 1b zeigt sie in einem vollständig befüllten Zustand bzw. welchen sie annimmt, wenn sie vollständig befüllt ist.

Die Spritze 1 weist einen Spritzenzylinder 2 und einen Kolben 5 auf.

Der Spritzenzylinder 2 weist einen kopfseitig hieran angeformten optionalen Luer-Konus 3 und einem am gegenüberliegenden Ende angeformten Spritzenflansch 4 auf.

Der Kolben 5 ist zumeist aus einem elastomeren Material und stopfenförmig ausgestaltet. Er befindet sich im Inneren des Spritzenzylinders 2 und ist mit einer Kolbenstange 6 verbunden, mittels welcher er innerhalb des Spritzenzylinders 2 axial verschiebbar ist.

Der Kolben 5 endet mit einer in Draufsicht zumeist ovalen oder runden Druckplatte 7. Sie ist in Fig. 1a und Fig. 1b von der Seite gezeigt.

In Fig. 1a steht der Kolben 5 in einer ersten Endlage B, in Fig. 1b in einer zweiten Endlage C. Die erste Endlage B nimmt der Kolben 5 an, wenn der Spritzeninhalt vollständig durch Verfahren des Kolbens 5 entleert ist. Die zweite Endlage C nimmt der Kolben 5 an, wenn die Spritze 1 maximal befüllt ist.

Der Verschiebeweg des Kolbens 5 innerhalb der Spritze 1 ist also durch die beiden Endlagen B und C begrenzt. Sein bei bestimmungsgemäßem Gebrauch der Spritze 1 einzuhaltender, maximaler Verschiebeweg, den es zur Vermeidung eines Herausziehens des Kolbens 5 aus dem Spritzenzylinder 2 und einer hiermit möglicherweise einhergehenden Kontaminationsgefahr nicht zu überschreiten gilt, wird hierin als hₘₐₓ bezeichnet.

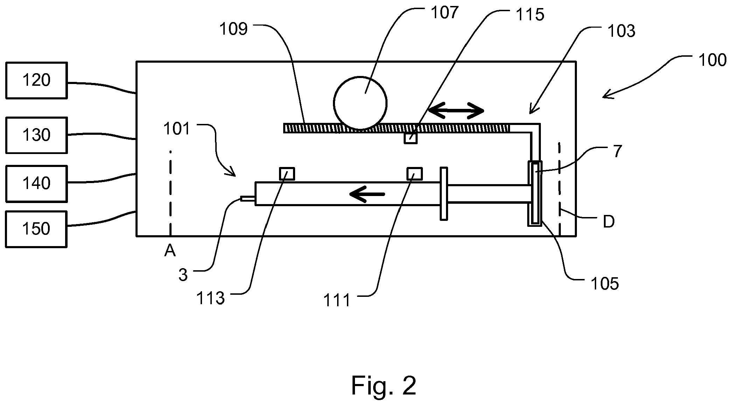

**Fig. 2** zeigt eine Ausführungsform einer erfindungsgemäßen Spritzenpumpe 100, welche eine Spritzenaufnahme mit einer Spritzenmulde 101 aufweist. Sie weist ferner einen verfahrbaren Schlitten 103 mit einem mit ihm gemeinsam verfahrbaren Knebel 105 auf, wobei der Knebel 105 relativ zum Schlitten 103 ortsfest ist oder ortsfester Teil hiervon ist.

Ein Motor 107 verfährt, z. B. mittels einer Spindel 109, den Schlitten 103 in Motorschritten. Die Richtung, in welcher sich der Verfahrweg des Schlittens 103 erstreckt, ist mittels Doppelpfeil gezeigt.

Das Verfahren des Schlittens 103 kommt nach links (bezogen auf Fig. 2), also zum Luer-Konus 3 hin, spätestens dann zum Erliegen, wenn ein erster Endanschlag A erreicht ist, welcher mittels Strichlinie angedeutet ist.

Das Verfahren des Schlittens 103 kommt nach rechts (bezogen auf Fig. 2), also zur Druckplatte 7 hin, spätestens dann zum Erliegen, wenn ein zweiter Endanschlag D erreicht ist, welcher ebenfalls mittels Strichlinie angedeutet ist.

Die Endanschläge A und D sind als mechanische Endstationen des Schlittens 103 zu verstehen: Ein Verfahren des Schlittens über diese Punkte A und D ist technisch nicht möglich, selbst wenn keine Spritze 1 in die Spritzenpumpe 100 eingelegt ist.

Die Endanschläge A und D liegen jeweils außerhalb der mit Bezug auf Fig. 1 beschriebenen ersten und zweiten Endlage B bzw. C.

An der Spritzenaufnahme sind ein erster Sensor 111 und ein zweiter Sensor 113 ortsfest mit z. B. einem Gehäuse der Spritzenpumpe 100 verbunden.

Der Schlitten 103 seinerseits kann einen Permanentmagnet 115 aufweisen, welcher dazu dienen kann, auf bekannte Weise im Wechselspiel mit dem ersten Sensor 111 und/oder dem zweiten Sensor 113, zu ermitteln, wenn der Schlitten 103 die Position des ersten Sensors 111 oder des zweiten Sensors 113 erreicht oder passiert. Der erste und der zweite Sensor 111 bzw. 113 sind hierzu als Hall-Sensoren ausgestaltet. Zum Ermitteln oder Auswerten der Signale, die von den Hall-Sensoren empfangen werden, erforderliche Auswertevorrichtungen sind vorgesehen.

Alternativ zu der Ausgestaltung der Sensoren 111 und 113 als Hall-Sensoren könnte einer oder beide dieser Sensoren auch als Lichtschranken ausgestaltet sein, wobei Kombinationen aus einem Hall-Sensor und einer Lichtschranke ebenfalls von der vorliegenden Erfindung umfasst sind.

Von der Erfindung ist ferner umfasst, dass sich die beiden Sensoren 111 und 113, oder auch nur ein beliebiger von ihnen, ortsfest zum Schlitten 103 gemeinsam mit diesem bewegen, wenn der Schlitten 103 verfahren wird. Der Permanentmagnet 115 kann sich in solchen Ausgestaltungen an der Spritzenaufnahme befinden.

Die Spritzenpumpe 100 weist ferner eine Zählvorrichtung 120, eine Steuervorrichtung 130, eine Speichervorrichtung 140 und eine Eingabevorrichtung 150 auf oder ist hiermit in Signalverbindung verbunden, wie in Fig. 2 angedeutet ist.

Fig. 2 zeigt die erfindungsgemäße Spritzenpumpe 100 mit in sie eingelegter Spritze 1. Diese gemeinsame Darstellung dient der besseren Erläuterung des Zusammenwirkens von Spritzenpumpe 100 und Spritze 1 im bestimmungsgemäßen Gebrauch der Spritzenpumpe 100 bzw. Spritze 1. Zum Kalibrieren muss die Spritze 1 hingegen nicht in die Spritzenpumpe 100 eingelegt sein.

Für die experimentelle Bestimmung des Offsets für den automatischen Kalibrierprozess wurde die Referenzspritze vorher in mehrere Spritzenpumpen desselben Typs eingelegt und der daraus ermittelte Mittelwert im Speicher hinterlegt. Dieser Prozess zur Bestimmung des Offsets zählt nicht zum bestimmungsgemäßen Gebrauch der Spritzenpumpe. Jedoch kann der Offset gemäß Schritt a) zum Kalibrieren hinterlegt oder eingegeben und entsprechend ausgelesen werden.

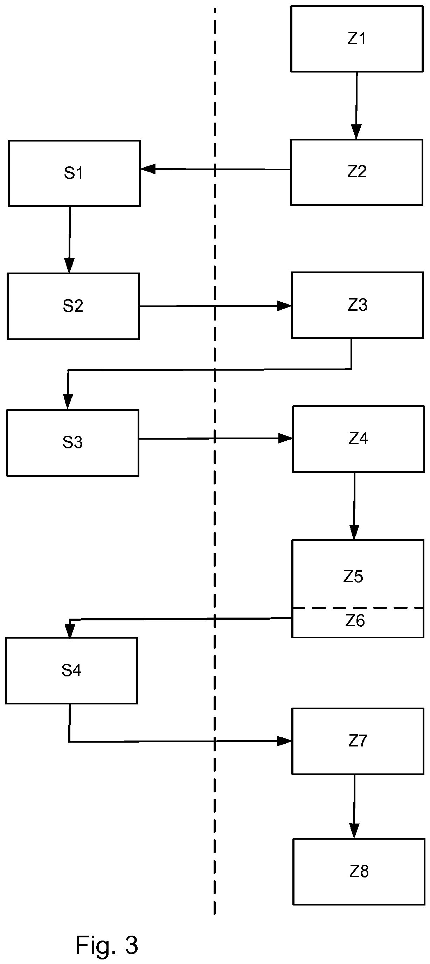

**Fig. 3** zeigt den Ablauf einer Ausführungsform des erfindungsgemäßen Verfahrens in einem Flussdiagramm, wobei die Schritte mit der Kennzeichnung "S" Schritte der erfindungsgemäßen Spritzenpumpe 100 den Schlitten betreffend und die Schritte mit der Kennzeichnung "Z" Schritte die Eingabevorrichtung 150 (oder mittels dieser), die Zählvorrichtung 120 oder die Speichervorrichtung 140 betreffend bezeichnen. Es gilt:
Schritt Z1 ist optional und betrifft das Eingeben wenigstens einer Information, welche einen konkreten Typ der Spritze 1 betrifft, über die Eingabevorrichtung 150. Hierzu kann vorstehender Offset zählen.
Schritt Z2 ist ebenfalls optional und betrifft das Auslesen der in der Speichervorrichtung 140 gespeicherten Information, welche einen konkreten Typ der Spritze 1 betrifft (z. B. hₘₐₓ).

Schritt S1 betrifft das Verfahren des Knebels 105 mittels des Schlittens 103 in eine Position zwischen der ersten Endlage B und der zweiten Endlage C, insbesondere in eine Position zwischen dem ersten Sensor 111 und dem zweiten Sensor 113, sofern diese Position nicht schon erreicht ist. Dies kann insbesondere mit einer ersten Geschwindigkeit erfolgen. Diese kann zwischen 100 µs/Mikroschritt und 1000 µs/Mikroschritt, vorzugsweise 500 µs/Mikroschritt, betragen.

Ein Mikroschritt ist eine Schrittweite des Pumpenmotors; der Begriff kann für ein Rotationsmaß oder für ein Linearmaß stehen.

Schritt S2 betrifft ein Verfahren des Knebels 105 mittels des Schlittens 103 derart, dass der Kolben bei eingelegter Spritze in die erste Endlage B gelangen würde oder bis ein vorbestimmter Abschnitt des Schlittens 103, beispielsweise der Permanentmagnet 115, an den ersten Endanschlag A gelangt ist, dies insbesondere mit einer zweiten Geschwindigkeit. Diese kann zwischen 1000 µs/Mikroschritt und 10000 µs/Mikroschritt, vorzugsweise 5000 µs/Mikroschritt, betragen. Alternativ kann die zweite Geschwindigkeit zwischen 1000 µs/Mikroschritt und 2000 µs/Mikroschritt, insbesondere 1400 µs/Mikroschritt, betragen.

Erreicht oder passiert hierbei gemäß Schritt Z3 ein vorbestimmter Abschnitt des Schlittens 103, z. B. der Permanentmagnet 115, die Position des zweiten Sensors 113, so werden die Motorschritte beim Passieren oder Erreichen dieser Position, beispielsweise von der Zählvorrichtung 120, als ein erster Zählwert ZW1 ermittelt und als Position des zweiten Sensors 113 gespeichert. Optional wird anschließend die Zählvorrichtung 120 auf "0" gesetzt.

Im Schritt S3 wird der Knebel 105 mittels des Schlittens 103 weiter in Richtung der ersten Endlage B bzw. des ersten Endanschlags A verfahren.

Damit wird in Schritt Z4 bei Erreichen der ersten Endlage B durch den Kolben 5 oder bei Erreichen des ersten Endanschlags A durch den Schlitten 103 ein, mittels der Zählvorrichtung 120 ermittelter, zweiter Zählwert ZW2 bestimmt. Dieser kann als Position der Endlage B oder als Position des Endanschlags A gespeichert werden.

Schritt Z5 betrifft das Errechnen eines Kalibrierwerts K2 des zweiten Sensors 113 aus den beiden vorigen Zählwerten ZW1 und ZW2, z. B. mittels der Zählvorrichtung 120 oder einer anderen hierfür programmierten Vorrichtung. Dieser Kalibrierwert K2 für den zweiten Sensor 113 wird mittels der Speichervorrichtung 140 gespeichert.

Schritt Z6 ist optional und betrifft das Zurücksetzten der Zählvorrichtung 120, z. B. auf "0".

In Schritt S4 wird der Knebel 105 mittels des Schlittens 103, insbesondere mit der o. g. zweiten oder mit einer dritten Geschwindigkeit, in Richtung der zweiten Endlage C verfahren. Die dritte Geschwindigkeit kann zwischen 10000 µs/Mikroschritt und 50000 µs/Mikroschritt, vorzugsweise 20000 µs/Mikroschritt, betragen.

Schritt Z7 betrifft das Ermitteln der Position des ersten Sensors 111 mittels der Zählvorrichtung 120, die ausgehend von der ersten Endlage B oder vom ersten Endanschlag A bis zum Erreichen oder bis zum Passieren des ersten Sensors 111 durch den vorbestimmten Abschnitt des Schlittens 103, z. B. den Permanentmagneten 115, durch einen dritten Zählwert ZW3 bestimmt ist, der optional, z. B. als Position des ersten Sensors 111, gespeichert wird.

In Schritt Z8 wird nun ein Kalibrierwert K1 für den ersten Sensor 111, beispielsweise aus dem ermittelten dritten Zählwert ZW3, bestimmt. In manchen Ausführungsformen kann dieser Kalibrierwert K1 zusätzlich unter Bezugnahme auf die bereits ermittelten und gespeicherten Längen und/oder Positionen errechnet werden. Dieser Kalibrierwert K1 für den ersten Sensor 111 wird ebenfalls mittels der Speichervorrichtung 140 gespeichert.

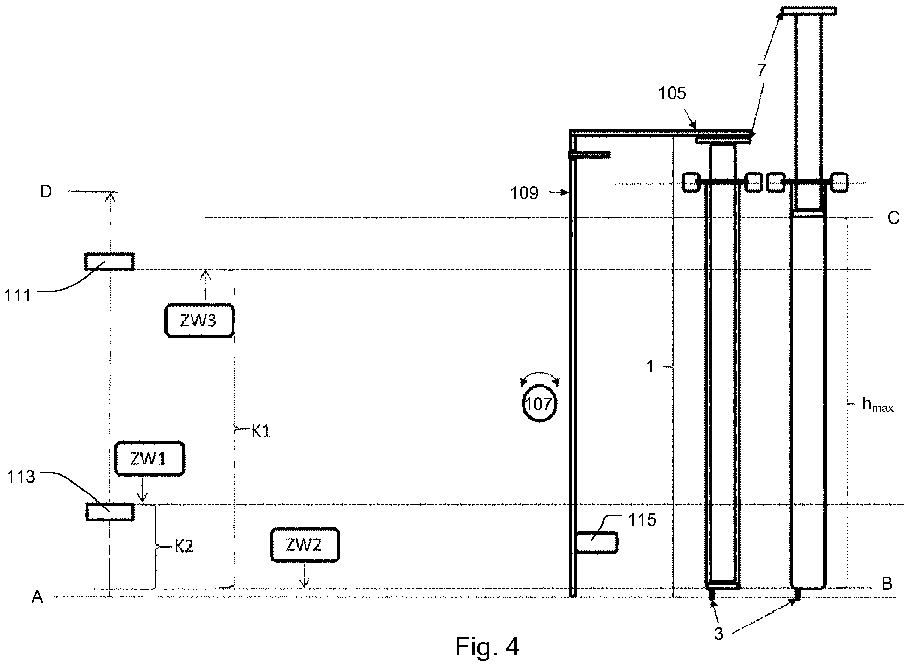

**Fig. 4** zeigt den Ablauf einer exemplarischen Ausführungsform des erfindungsgemäßen Verfahrens in einer Übersicht.

Die Übersicht veranschaulicht weiter die ermittelten Zählwerte ZW1, ZW2 und ZW3. Diese werden jeweils ermittelt, wenn der hier exemplarische Permanentmagnet 115 in der durch den jeweiligen Pfeil angegebenen Richtung den entsprechenden Sensor 111, 113 erreicht und/oder passiert bzw. die erste Endlage B erreicht. Diese Zählwerte ZW1, ZW2 und ZW3 können zum Ermitteln der Kalibrierwerte K1 und K2, welche die Position der Hall-Sensoren zum ersten Endanschlag A (bzw. zur ersten Endlage B) angeben, verwendet werden.

Alternativ ist es möglich, die Zählvorrichtung 120 beim Erreichen der jeweiligen Positionen auf Null zu setzen und daraufhin die ermittelten Zählwerte direkt als Kalibrierwerte zu übernehmen.

### Bezugszeichenliste

- 1: Spritze
- 2: Spritzenzylinder
- 3: Luer-Konus
- 4: Spritzenflansch
- 5: Kolben

- 6: Kolbenstange
- 7: Druckplatte

- 100: Spritzenpumpe
- 101: Spritzenmulde
- 103: Schlitten
- 105: Knebel
- 107: Motor
- 109: Spindel
- 111: erster Sensor
- 113: zweiter Sensor
- 115: Permanentmagnet
- 120: Zählvorrichtung
- 130: Steuervorrichtung
- 140: Speichervorrichtung
- 150: Eingabevorrichtung

- A: erster Endanschlag
- B: erste Endlage
- C: zweite Endlage
- D: zweiter Endanschlag
- Sx: x-ter Verfahrensschritt auf Seite der Spritzenpumpe
- Zx: x-ter Verfahrensschritt auf Seite der Eingabe- oder Speicher- oder Zählvorrichtung
- K1: Kalibrierwert des ersten Sensors 111
- K2: Kalibrierwert des zweiten Sensors 113
- ZW1: erster Zählwert
- ZW2: zweiter Zählwert
- ZW3: dritter Zählwert
- hₘₐₓ: maximaler Verschiebeweg des Kolbens oder der Kolbenstange innerhalb des Spritzenzylinders bei bestimmungsgemäßer Verwendung der Spritze; der maximale Verschiebeweg ist derart festgelegt, dass ein unerwünschtes Herausziehen des Kolbens aus dem Zylinder vermieden wird

## Patentansprüche

1. Verfahren zum Kalibrieren einer medizinischen Spritzenpumpe (100), welche aufweist
- eine Spritzenaufnahme zum Aufnehmen einer Spritze (1), wobei die Spritze (1) einen Spritzenzylinder (2) und einen hierin zwischen einer ersten Endlage (B) und einer zweiten Endlage (C) verfahrbaren Kolben (5) aufweist, wobei der Kolben (5) mit einer Kolbenstange (6) verbunden ist;
- einen in Motorschritten verfahrbaren Schlitten (103) mit einem Knebel (105) zum Aufnehmen eines Abschnitts der Kolbenstange (6) oder zu einem Verbinden hiermit, wobei ein Abschnitt des Schlittens (103) maximal zwischen einem ersten Endanschlag (A) und einem zweiten Endanschlag (D) relativ zur Spritzenaufnahme verfahrbar angeordnet ist, und wobei die Spritzenpumpe (100) konfiguriert ist, den Schlitten (103) in Motorschritten zu verfahren;
- eine Zählvorrichtung (120) zum Zählen der Anzahl von Motorschritten, um welche der Schlitten (103) verfahren wird;
- einen ersten Sensor (111) und einen zweiten Sensor (113), beide angeordnet entlang eines Verschiebewegs des Kolbens (5) oder eines Verfahrwegs des Schlittens (103);
- eine Steuervorrichtung (130), konfiguriert zum Verfahren des Schlittens (103) entsprechend Steueranweisungen;
- eine Speichervorrichtung (140);
- eine Eingabevorrichtung (150) zum Eingeben von Steueranweisungen und/oder anderen Daten durch einen Anwender;
**dadurch gekennzeichnet, dass** das Verfahren folgende Schritte umfasst:
a) optional: Eingeben wenigstens einer Information einen konkreten Typ der Spritze (1) betreffend mittels der Eingabevorrichtung (150) und Speichern dieser Information mittels der Speichervorrichtung (140);
b) Verfahren eines Abschnitts des Schlittens, mittels einer Bewegung des Schlittens (103) in eine Position zwischen der ersten Endlage (B) und der zweiten Endlage (C), insbesondere in eine Position zwischen dem ersten Sensor (111) und dem zweiten Sensor (113), insbesondere mit einer ersten Geschwindigkeit;
c) Verfahren des Knebels (105) mittels des Schlittens (103) derart, dass der Kolben (5) bei eingelegter Spritze in die erste Endlage (B) gelangen würde, oder bis ein vorbestimmter Abschnitt des Schlittens (103), insbesondere ein Permanentmagnet (115), an den ersten Endanschlag (A) gelangt ist, insbesondere mit einer zweiten Geschwindigkeit,
wobei, wenn ein vorbestimmter Abschnitt des Schlittens (103) die Position des zweiten Sensors (113) erreicht oder passiert, die für oder an dieser Position von der Zählvorrichtung (120) ausgegebene Anzahl an Motorschritten als ein erster Zählwert (ZW1) ermittelt wird, und wobei bei Erreichen der ersten Endlage (B) durch den Kolben (5) oder bei Erreichen des ersten Endanschlags (A) durch den Abschnitt des Schlittens (103) die bis zum Erreichen der Position der ersten Endlage (B) oder des ersten Endanschlags (A) mittels der Zählvorrichtung (120) gezählten Motorschritte als ein zweiter Zählwert (ZW2) ermittelt werden;
d) Errechnen eines Kalibrierwerts (K2) des zweiten Sensors (113) aus dem ersten Zählwert (ZW1) und dem zweiten Zählwert (ZW2) und Speichern dieses Kalibrierwerts (K2) für den zweiten Sensor (113) mittels der Speichervorrichtung (140);
e) optional: Zurücksetzten der Zählvorrichtung, z. B. auf "0" oder einen vorbestimmten Startwert;
f) Verfahren des Knebels (105), insbesondere mit der zweiten oder mit einer dritten Geschwindigkeit, in Richtung der zweiten Endlage (C);
g) Ermitteln der Anzahl an Motorschritten, welche die Zählvorrichtung (120) bei Erreichen der Position des ersten Sensors (111) oder Passieren mittels des vorbestimmten Abschnitts des Schlittens (103) ausgibt als dritten Zählwert (ZW3);
h) Errechnen eines Kalibrierwerts (K1) des ersten Sensors (111) aus dem dritten Zählwert (ZW3) und optional aus wenigstens einem weiteren Wert aus der Gruppe, die aus dem ersten Zählwert (ZW1), dem zweiten Zählwert (ZW2) und dem Kalibrierwert (K2) des zweiten Sensors (113) besteht, und Speichern dieses Kalibrierwerts (K1) für den ersten Sensor (111) mittels der Speichervorrichtung (140).

2. Verfahren nach Anspruch 1, wobei zum Verfahren gemäß Schritt b) der Abschnitt des Schlittens (103) zunächst in Richtung des ersten Endanschlags (A) verfahren wird und wobei das Verfahren des Abschnitts des Schlittens (103) mit der zweiten Geschwindigkeit fortgesetzt wird, nachdem entweder die Position des zweiten Sensors (113) erreicht ist oder der erste Endanschlag (A) erreicht ist,
wobei der Abschnitt des Schlittens (103) nach Erreichen der Position des zweiten Sensors (113) oder nach Erreichen des ersten Endanschlags (A) automatisch um eine vorbestimmte Anzahl an Motorschritten in Richtung des zweiten Endanschlags (D) verfahren wird, bevor er anschließend gemäß Schritt c) weiter verfahren wird.

3. Verfahren nach einem der vorangegangenen Ansprüche, wobei die ermittelte Anzahl an Motorschritten mit einem Kompensationswert verrechnet wird und auf diese Weise kompensiert in die Berechnung der Kalibrierwerte (K1, K2) von erstem Sensor (111) und/oder zweitem Sensor (113) gemäß der Schritte d) und g) eingehen.

4. Verfahren nach dem vorangegangenen Anspruch, wobei die auf diese Weise korrigierten Kalibrierwerte (K1, K2) des ersten Sensors (111) und/oder des zweiten Sensors (113) auf Plausibilität überprüft werden.

5. Verfahren nach einem der vorangegangenen Ansprüche, wobei der erste Sensor (111) und/oder der zweite Sensor (113) ein Magnetfeldsensor, insbesondere ein Hall-Sensor, oder ein optoelektronischer Sensor, insbesondere eine Lichtschranke, und/oder Teil einer Widerstandspiste sind, die nach dem Prinzip eines Potentiometers arbeitet.

6. Verfahren nach einem der vorangegangenen Ansprüche, wobei der Schlitten (103) einen in unveränderlichem Abstand zum Abschnitt des Schlittens (103) verfahrbaren Permanentmagnet (115) aufweist.

7. Verfahren nach einem der vorangegangenen Ansprüche, wobei die Information den konkreten Spritzentyp betreffend wenigstens den Abstand (hₘₐₓ) zwischen erster Endlage (B) und zweiter Endlage (C) und/oder einen Korrekturfaktor und/oder einen Offset umfasst.

8. Medizinische Spritzenpumpe (100), welche aufweist
- eine Spritzenaufnahme zum Aufnehmen einer Spritze (1), die einen Spritzenzylinder (2) und einen hierin zwischen einer ersten Endlage (B) und einer zweiten Endlage (C) verfahrbaren Kolben (5) aufweist, wobei der Kolben (5) mit einer Kolbenstange (6) verbunden ist;
- einen in Motorschritten verfahrbaren Schlitten (103) mit einem Knebel (105) zum Aufnehmen eines Abschnitts der Kolbenstange (6) oder einem Verbinden hiermit, wobei ein Abschnitt des Schlittens (103) maximal zwischen einem ersten Endanschlag (A) und einem zweiten Endanschlag (D) relativ zur Spritzenaufnahme verfahrbar angeordnet ist, und wobei die Spritzenpumpe (100) konfiguriert ist, den Schlitten (103) in Motorschritten zu verfahren;
- eine Zählvorrichtung (120) zum Zählen der Anzahl von Motorschritten, um welche der Schlitten (103) verfahren wird;
- einen ersten Sensor (111) und einen zweiten Sensor (113), angeordnet entlang eines Verschiebewegs des Kolbens (5) oder eines Verfahrwegs des Schlittens (103);
- eine Steuervorrichtung (130), konfiguriert zum Verfahren des Schlittens (103) entsprechend Steueranweisungen;
- eine Speichervorrichtung (140);
- eine Eingabevorrichtung (150) zum Eingeben von Steueranweisungen und/oder anderen Daten durch einen Anwender;
**dadurch gekennzeichnet, dass** die Steuervorrichtung (130) konfiguriert und/oder vorbereitet ist, ein Verfahren zum Kalibrieren auszuführen, welches folgende Schritte umfasst:
a) optional: Eingeben wenigstens einer Information einen konkreten Typ der Spritze (1) betreffend mittels der Eingabevorrichtung (150) und Speichern dieser Information mittels der Speichervorrichtung (140);
b) Verfahren eines Abschnitts des Schlittens, mittels einer Bewegung des Schlittens (103) in eine Position zwischen der ersten Endlage (B) und der zweiten Endlage (C), insbesondere in eine Position zwischen dem ersten Sensor (111) und dem zweiten Sensor (113), insbesondere mit einer ersten Geschwindigkeit;
c) Verfahren des Knebels (105) mittels des Schlittens (103) derart, dass der Kolben (5) bei eingelegter Spritze in die erste Endlage (B) gelangen würde, oder bis ein vorbestimmter Abschnitt des Schlittens (103), insbesondere ein Permanentmagnet (115), an den ersten Endanschlag (A) gelangt ist, insbesondere mit einer zweiten Geschwindigkeit,
wobei, wenn ein vorbestimmter Abschnitt des Schlittens (103) die Position des zweiten Sensors (113) erreicht oder passiert, die für oder an dieser Position von der Zählvorrichtung (120) angegebenen Anzahl an Motorschritten als ein erster Zählwert (ZW1) ermittelt wird, und wobei bei Erreichen der ersten Endlage (B) durch den Kolben (5) oder bei Erreichen des ersten Endanschlags (A) durch den Abschnitt des Schlittens (103) die bis zum Erreichen der Position der ersten Endlage (B) oder des ersten Endanschlags (A) mittels der Zählvorrichtung (120) gezählten Motorschritte als ein zweiter Zählwert (ZW2) ermittelt werden;
d) Errechnen eines Kalibrierwerts (K2) des zweiten Sensors (113) aus dem ersten Zählwert (ZW1) und dem zweitem Zählwert (ZW2) und Speichern dieses Kalibrierwerts (K2) für den zweiten Sensor (113) mittels der Speichervorrichtung (140);
e) optional: Zurücksetzten der Zählvorrichtung, z. B. auf "0" oder einen vorbestimmten Startwert;
f) Verfahren des Knebels (105), insbesondere mit der zweiten oder mit einer dritten Geschwindigkeit, in Richtung der zweiten Endlage (C);
g) Ermitteln der Anzahl an Motorschritten, welche die Zählvorrichtung (120) bei Erreichen der Position des ersten Sensors (111) oder Passieren mittels des vorbestimmten Abschnitts des Schlittens (103) ausgibt als dritten Zählwert (ZW3);
h) Errechnen eines Kalibrierwerts (K1) des ersten Sensors (111) aus dem dritten Zählwert (ZW3) und optional aus wenigstens einem weiteren Wert aus der Gruppe, die aus dem ersten Zählwert (ZW1), dem zweiten Zählwert (ZW2) und dem Kalibrierwert (K2) des zweiten Sensors (113) besteht, und Speichern dieses Kalibrierwerts (K1) für den ersten Sensor (111) mittels der Speichervorrichtung (140).

9. Medizinische Spritzenpumpe (100) nach Anspruch 8, wobei zum Verfahren gemäß Schritt b) der Abschnitt des Schlittens (103) zunächst in Richtung des ersten Endanschlags (A) verfahren wird, und das Verfahren des Abschnitts des Schlittens (103) mit der zweiten Geschwindigkeit fortgesetzt wird, nachdem entweder die Position des zweiten Sensors (113) erreicht ist oder der erste Endanschlag (A) erreicht ist,
wobei der Abschnitt des Schlittens (103) nach Erreichen des zweiten Sensors (113) oder nach Erreichen des ersten Endanschlags (A) automatisch um eine vorbestimmte Anzahl an Motorschritten in Richtung des zweiten Endanschlags (D) verfahren wird, bevor er anschließend wie in Schritt c) weiter verfahren wird.

10. Medizinische Spritzenpumpe (100) nach wenigstens einem der Ansprüche 8 bis 9, wobei die ermittelte Anzahl an Motorschritten mit einem Kompensationswert verrechnet wird und auf diese Weise kompensiert in die Berechnung der Kalibrierwerte (K1, K2) von erstem Sensor (111) und/oder zweitem Sensor (113) gemäß der Schritte d) und g) eingeht.

11. Medizinische Spritzenpumpe (100) nach dem vorangegangenen Anspruch, wobei die auf diese Weise kompensierten Kalibrierwerte (K1, K2) des ersten Sensors (111) und/oder des zweiten Sensors (113) auf Plausibilität überprüft werden.

12. Medizinische Spritzenpumpe (100) nach wenigstens einem der Ansprüche 8 bis 11, wobei der erste Sensor (111) und/oder der zweite Sensor (113) ein Magnetfeldsensor, insbesondere ein Hall-Sensor, oder ein optoelektronischer Sensor, insbesondere eine Lichtschranke, ist.

13. Medizinische Spritzenpumpe (100) nach wenigstens einem der Ansprüche 8 bis 12, wobei der Schlitten (103) einen in unveränderlichem Abstand mit dem Abschnitt des Schlittens (103) verfahrbaren Permanentmagnet (115) aufweist.

14. Medizinische Spritzenpumpe (100) nach wenigstens einem der Ansprüche 8 bis 13, wobei die Information den konkreten Spritzentyp betreffend wenigstens den Abstand (hₘₐₓ) zwischen erster Endlage (B) und zweiter Endlage (C) und/oder einen Korrekturfaktor und/oder einen Offset umfasst.

15. Medizinische Spritzenpumpe (100) nach wenigstens einem der Ansprüche 8 bis 14, ausgestaltet als Heparinspritzenpumpe.

16. Blutbehandlungsvorrichtung, aufweisend oder in Signalkommunikation verbunden mit einer medizinischen Spritzenpumpe (100) nach einem der Ansprüche 8 bis 15.

17. Blutbehandlungsvorrichtung nach Anspruch 16, ausgestaltet als Peritonealdialysevorrichtung, Hämodialysevorrichtung, Hämofiltrationsvorrichtung oder als Hämodiafiltrationsvorrichtung, insbesondere als eine Vorrichtung für die chronische Nierenersatztherapie oder für die kontinuierliche Nierenersatztherapie (CRRT = *continuous renal replacement therapy).*

18. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die maschinellen Schritte eines erfindungsgemäßen Verfahrens nach einem der Ansprüche 1 bis 7 veranlasst werden.

19. Computerprogramm-Produkt, als Signalwelle oder mit einem auf einem maschinenlesbaren Träger gespeicherten Programmcode, zur Veranlassung der maschinellen Schritte des erfindungsgemäßen Verfahrens gemäß einem der Ansprüche 1 bis 7, wenn das Computerprogramm-Produkt auf einem Rechner abläuft.

## Claims

1. A method of calibrating a medical syringe pump (100) comprising:
- a syringe receptacle for receiving a syringe (1), wherein the syringe (1) comprises a syringe cylinder (2) and a plunger (5) being displaceable therein between a first end position (B) and a second end position (C), the plunger (5) being connected to a plunger rod (6);
- a slide (103) displaceable in motor steps having a toggle (105) for receiving a section of the plunger rod (6) or for connecting it thereto, wherein a section of the slide (103) is arranged to be displaceable maximally between a first end stop (A) and a second end stop (D) relative to the syringe receptacle, and wherein the syringe pump (100) is configured to displace the slide (103) in motor steps;
- a counting device (120) for counting the number of motor steps by which the slide (103) is being displaced;
- a first sensor (111) and a second sensor (113), both arranged along a shifting path of the plunger (5) or along a displacement path of the slide (103);
- a control device (130), configured to displace the slide (103) according to control instructions;
- a storage device (140);
- an input device (150) for entering control instructions and/or other data by a user;
**characterized in that** the method comprises the following steps:
a) optionally: entering at least one information concerning a specific type of syringe (1) by means of the input device (150) and storing this information by means of the storage device (140);
b) displacing a section of the slide, by moving the slide (103) into a position between the first end position (B) and the second end position (C), in particular into a position between the first sensor (111) and the second sensor (113), in particular at a first speed;
c) displacing the toggle (105) by means of the slide (103) such that the plunger (5) would reach the first end position (B) when the syringe is inserted, or until a predetermined section of the slide (103), in particular a permanent magnet (115), has reached the first end stop (A), in particular at a second speed;
wherein, when a predetermined section of the slide (103) reaches or passes the position of the second sensor (113), the number of motor steps output by the counting device (120) for or at this position is determined as a first count value (ZW1), and wherein, when the plunger (5) reaches the first end position (B) or when the section of the slide (103) reaches the first end stop (A), the motor steps, counted by means of the counting device (120) until reaching the position of the first end position (B) or the first end stop (A) are determined as a second count value (ZW2);
d) calculating a calibration value (K2) of the second sensor (113) from the first count value (ZW1) and the second count value (ZW2) and storing this calibration value (K2) for the second sensor (113) by means of the storage device (140);
e) optionally: resetting the counting device, e.g. to "0" or to a predetermined start value;
f) displacing the toggle (105), in particular at the second or at a third speed, in direction of the second end position (C);
g) determining the number of motor steps, which the counting device (120) outputs as third count value (ZW3), when reaching or passing the position of the first sensor (111) by means of the predetermined section of the slide (103) ;
h) calculating a calibration value (K1) of the first sensor (111) from the third count value (ZW3) and optionally from at least one further value from the group consisting of the first count value (ZW1), the second count value (ZW2) and the calibration value (K2) of the second sensor (113), and storing this calibration value (K1) for the first sensor (111) by means of the storage device (140).

2. The method according to claim 1, wherein to proceed according to step b), the section of the slide (103) is first displaced in direction of the first end stop (A), and wherein the displacing of the section of the slide (103) at the second speed continues after reaching either the position of the second sensor (113) or the first end stop (A),
wherein, after reaching the position of the second sensor (113), or after reaching the first end stop (A), the section of the slide (103) is automatically displaced by a predetermined number of motor steps in direction of the second end stop (D), before being then further displaced according to step c) .

3. The method according to anyone of the preceding claims, wherein the determined number of motor steps is offset with a compensation value and thus compensated in the calculation of the calibration values (K1, K2) of the first sensor (111) and/or of the second sensor (113) according to steps d) and g).

4. The method according to the preceding claim, wherein the thus obtained compensated calibration values (K1, K2) of the first sensor (111) and/or of the second sensor (113) are checked for plausibility.

5. The method according to anyone of the preceding claims, wherein the first sensor (111) and/or the second sensor (113) is/are a magnetic field sensor, in particular a Hall sensor, or an optoelectronic sensor, in particular a light barrier, and/or part of a resistance track operating according to the principle of a potentiometer.

6. The method according to anyone of the preceding claims, wherein the slide (103) comprises a permanent magnet (115) being displaceable at a constant distance from the section of the slide (103).

7. The method according to anyone of the preceding claims, wherein the information concerning the specific syringe type includes at least the distance (hₘₐₓ) between the first end (B) and the second end position (C) and/or a correction factor and/or an offset.

8. The medical syringe pump (100) which comprises:
- a syringe receptacle for receiving a syringe (1), comprising a syringe cylinder (2) and a plunger (5) displaceable therein between a first end position (B) and a second end position (C), the plunger (5) being connected to a plunger rod (6);
- a slide (103) displaceable in motor steps having a toggle (105) for receiving a section of the plunger rod (6) or for connecting it thereto, wherein a section of the slide (103) is arranged to be displaceable maximally between a first end stop (A) and a second end stop (D) relative to the syringe receptacle, and wherein the syringe pump (100) is configured to displace the slide (103) in motor steps;
- a counting device (120) for counting the number of motor steps by which the slide (103) is being displaced;
- a first sensor (111) and a second sensor (113) arranged along a shifting path of the plunger (5) or along a displacement path of the slide (103);
- a control device (130) configured for displacing the slide (103) according to control instructions;
- a storage device (140);
- an input device (150) for entering control instructions and/or other data by a user;
**characterized in that** the control device (130) is configured and/or prepared for executing a method of calibrating, which includes the following steps:
a) optionally: entering at least one information concerning a specific type of syringe (1) by means of the input device (150) and storing this information by means of the storage device (140);
b) displacing a section of the slide, by moving the slide (103) into a position between the first end position (B) and the second end position (C), in particular into a position between the first sensor (111) and the second sensor (113), in particular at a first speed;
c) displacing the toggle (105) by means of the slide (103) such that the plunger (5) would reach the first end position (B) when the syringe is inserted, or until a predetermined section of the slide (103), in particular a permanent magnet (115), has reached the first end stop (A), in particular at a second speed;
wherein, when a predetermined section of the slide (103) reaches or passes the position of the second sensor (113), the number of motor steps output by the counting device (120) for or at this position is determined as a first count value (ZW1), and wherein, when the plunger (5) reaches the first end position (B) or when the section of the slide (103) reaches the first end stop (A), the motor steps, counted by means of the counting device (120), until reaching the position of the first end position (B) or the first end stop (A), are determined as a second count value (ZW2);
d) calculating a calibration value (K2) of the second sensor (113) from the first count value (ZW1) and the second count value (ZW2) and storing this calibration value (K2) for the second sensor (113) by means of the storage device (140);
e) optionally: resetting the counting device, e.g. to "0" or to a predetermined start value;
f) displacing the toggle (105), in particular at the second or at a third speed, in direction of the second end position (C);
g) determining the number of motor steps, which the counting device (120) outputs as third count value (ZW3), when reaching or passing the position of the first sensor (111) by means of the predetermined section of the slide (103) ;
h) calculating a calibration value (K1) of the first sensor (111) from the third count value (ZW3) and optionally from at least one further value from the group consisting of the first count value (ZW1), the second count value (ZW2) and the calibration value (K2) of the second sensor (113), and storing this calibration value (K1) for the first sensor (111) by means of the storage device (140).

9. The medical syringe pump (100) according to claim 8, wherein to proceed according to step b), the section of the slide (103) is first displaced in direction of the first end stop (A), and the displacing of the section of the slide (103) at the second speed continues after reaching either the position of the second sensor (113) or the first end stop (A),
wherein, after reaching the position of the second sensor (113), or after reaching the first end stop (A), the section of the slide (103) is automatically displaced by a predetermined number of motor steps in direction of the second end stop (D) before being then further displaced according to step c) .

10. The medical syringe pump (100) according to at least one of the claims 8 to 9, wherein the determined number of motor steps is offset with a compensation value and thus compensated in the calculation of the calibration values (K1, K2) of the first sensor (111) and/or of the second sensor (113) according to steps d) and g).

11. The medical syringe pump (100) according to the preceding claim, wherein the thus compensated calibration values (K1, K2) of the first sensor (111) and/or of the second sensor (113) are checked for plausibility.

12. The medical syringe pump (100) according to at least one of the claims 8 to 11, wherein the first sensor (111) and/or the second sensor (113) is/are a magnetic field sensor, in particular a Hall sensor, or an optoelectronic sensor, in particular a light barrier.

13. The medical syringe pump (100) according to at least one of the claims 8 to 12, wherein the slide (103) comprises a permanent magnet (115) being displaceable at a constant distance from the section of the slide (103).

14. The medical syringe pump (100) according to at least one of the claims 8 to 13, wherein the information concerning the specific syringe type includes at least the distance (hₘₐₓ) between the first end (B) and the second end position (C) and/or a correction factor and/or an offset.

15. The medical syringe pump (100) according to at least one of the claims 8 to 14, designed as heparin syringe pump.

16. A blood treatment apparatus, comprising or being connected in signal communication with a medical syringe pump (100) according to anyone of claims 8 to 15.

17. The blood treatment apparatus according to claim 16, designed as a peritoneal dialysis apparatus, hemodialysis apparatus, hemofiltration apparatus or hemodiafiltration apparatus, in particular as an apparatus for chronic renal replacement therapy or for continuous renal replacement therapy (CRRT).

18. A digital storage medium, in particular in the form of a floppy disk, CD or DVD or EPROM, with electronically readable control signals, configured to interact with a programmable computer system so as to prompt the automatic steps of the method based on the invention according to anyone of claims 1 to 7.

19. A computer program product, as a signal wave or having a program code stored on a machine-readable carrier, for prompting the automatic steps of the method based on the invention according to anyone of claims 1 to 7, when the computer program product is running on a computer.

## Revendications

1. Un procédé de calibrage d'un pousse-seringue médical (100) comprenant:
- un réceptacle à seringue pour recevoir une seringue (1), où la seringue (1) comprend un cylindre de seringue (2) ainsi qu'un piston (5) déplaçable à l'intérieur de celui-ci entre une première position terminale (B) et une seconde position terminale (C), le piston (5) étant relié à une tige de piston (6);
- un coulisseau (103) déplaçable par pas motorisés ayant un élément (105) pour recevoir une section de la tige de piston (6), ou s'y relier, où une section du coulisseau (103) est agencée de manière à pouvoir se déplacer au maximum entre une première butée d'arrêt (A) et une seconde butée d'arrêt (D) par rapport au réceptacle à seringue, et où le pousse-seringue (100) est configuré de manière à déplacer le coulisseau (103) par pas motorisés;
- un dispositif de comptage (120) pour compter le nombre de pas motorisés dont le coulisseau (103) est déplacé;
- un premier capteur (111) et un second capteur (113), tous deux agencés le long d'une trajectoire de déplacement du piston (5) ou d'une trajectoire de déplacement du coulisseau (103);
- un dispositif de commande (130) configuré pour déplacer le coulisseau (103) selon des consignes de commande;
- un dispositif d'enregistrement (140);
- un dispositif de saisie (150) pour saisir des instructions de commande et/ou d'autres données par un utilisateur;
**caractérisé en ce que** le procédé comprend les étapes suivantes:
a) éventuellement: la saisie d'au moins une information relative à un type spécifique de seringue (1) au moyen du dispositif de saisie (150) et l'enregistrement de cette information au moyen du dispositif
d'enregistrement (140);
b) le déplacement d'une section du coulisseau, au moyen d'un mouvement du coulisseau (103) vers une position située entre la première position terminale (B) et la seconde position terminale (C), notamment vers une position située entre le premier capteur (111) et le second capteur (113), notamment à une première vitesse;
c) le déplacement de l'élément (105) au moyen du coulisseau (103) de manière à ce que le piston (5) atteigne, lorsque la seringue est insérée, la première position terminale (B), ou jusqu'à ce qu'une section prédéterminée du coulisseau (103), notamment un aimant permanent (115), ait atteint la première butée d'arrêt (A), notamment à une seconde vitesse;
où, lorsqu'une section prédéterminée du coulisseau (103) atteint ou dépasse la position du second capteur (113), le nombre de pas motorisés émis par le dispositif de comptage (120) pour ou à cette position est considéré comme une première valeur de comptage (ZW1),
et où, lorsque le piston (5) atteint la première position terminale (B) ou lorsque la section du coulisseau (103) atteint la première butée d'arrêt (A), les pas motorisés, comptés au moyen du dispositif de comptage (120) jusqu'à ce que soit atteinte la position de la première position terminale (B) ou la première butée d'arrêt (A), sont considérés comme une seconde valeur de comptage (ZW2);
d) le calcul d'une valeur de calibrage (K2) du second capteur (113) à partir de la première valeur de comptage (ZW1) et de la seconde valeur de comptage (ZW2) et l' enregistrement de cette valeur de calibrage (K2) pour le second capteur (113) au moyen du dispositif d'enregistrement (140);
e) éventuellement: la réinitialisation du dispositif de comptage, par exemple à "0" ou à une valeur de départ prédéterminée;
f) le déplacement de l'élément (105), notamment à la seconde ou à une troisième vitesse, en direction de la seconde position terminale (C);
g) la détermination du nombre de pas motorisés que le dispositif de comptage (120) délivre en tant que troisième valeur de comptage (ZW3), lorsqu'il atteint ou dépasse la position du premier capteur (111) au moyen de la section prédéterminée du coulisseau (103);
h) le calcul d'une valeur de calibrage (K1) du premier capteur (111) à partir de la troisième valeur de comptage (ZW3) et éventuellement d'au moins une autre valeur du groupe constitué de la première valeur de comptage (ZW1), de la seconde valeur de comptage (ZW2) ainsi que de la valeur de calibrage (K2) du second capteur (113), et l'enregistrement de cette valeur de calibrage (K1) pour le premier capteur (111) au moyen du dispositif d'enregistrement (140).

2. Le procédé selon la première revendication, où lors du déplacement selon l'étape b), la section du coulisseau (103) est d'abord déplacée en direction de la première butée d'arrêt (A), et où la section du coulisseau (103) continue à se déplacer à la seconde vitesse après avoir atteint soit la position du second capteur (113), soit la première butée d'arrêt (A);
où, après avoir atteint la position du second capteur (113) ou après avoir atteint la première butée d'arrêt (A), la section du coulisseau (103) est automatiquement déplacée d'un nombre prédéterminé de pas motorisés en direction de la seconde butée d'arrêt (D) avant d'être ensuite déplacée selon l'étape c) .

3. Le procédé selon l'une quelconque des revendications précédentes, où le nombre déterminé de pas motorisés est équilibré par une valeur de compensation et est ainsi compensé dans le calcul des valeurs de calibrage (K1, K2) du premier capteur (111) et/ou du second capteur (113) selon les étapes d) et g).

4. Le procédé selon la revendication précédente, où les valeurs de calibrage (K1, K2) du premier capteur (111) et/ou du second capteur (113) ainsi compensées sont vérifiées quant à leur plausibilité.

5. Le procédé selon l'une quelconque des revendications précédentes, où le premier capteur (111) et/ou le second capteur (113) est/sont un capteur de champ magnétique, notamment un capteur à effet Hall, ou un capteur optoélectronique, notamment une barrière lumineuse, et/ou une partie d'une piste de résistance fonctionnant selon le principe d'un potentiomètre.

6. Le procédé selon l'une quelconque des revendications précédentes, où le coulisseau (103) comprend un aimant permanent (115) déplaçable à une distance constante de la partie du coulisseau (103).

7. Le procédé selon l'une quelconque des revendications précédentes, où l'information concernant le type spécifique de seringue inclut au moins la distance (hₘₐₓ) entre la première position terminale (B) et la seconde position terminale (C) et/ou un facteur de correction et/ou un décalage.

8. Un pousse-seringue médical (100) qui comprend:
- un réceptacle à seringue pour recevoir une seringue (1), comprenant un cylindre de seringue (2) ainsi qu'un piston (5) déplaçable à l'intérieur de celui-ci entre une première position terminale (B) et une seconde position terminale (C), le piston (5) étant relié à une tige de piston (6);
- un coulisseau (103) déplaçable par pas motorisés ayant un élément (105) pour recevoir une section de la tige de piston (6) ou pour s'y relier, où une section du coulisseau (103) est agencée de manière à pouvoir se déplacer au maximum entre une première butée d'arrêt (A) et une seconde butée d'arrêt (D) par rapport au réceptacle à seringue, et où le pousse-seringue (100) est configuré pour déplacer le coulisseau (103) par pas motorisés;
- un dispositif de comptage (120) pour compter le nombre de pas motorisés dont le coulisseau (103) est déplacé;
- un premier capteur (111) ainsi qu'un second capteur (113) agencés le long d'une trajectoire de déplacement du piston (5) ou d'une trajectoire de déplacement du coulisseau (103);
- un dispositif de commande (130), pour déplacer le coulisseau (103) selon des instructions de commande;
- un dispositif d'enregistrement (140);
- un dispositif de saisie (150) pour entrer des instructions de commande et/ou d'autres données par un utilisateur;
**caractérisé en ce que** le dispositif de commande (130) est configuré et/ou préparé, de façon à exécuter un procédé de calibrage qui inclut les étapes suivantes:
a) éventuellement: la saisie d'au moins une information relative à un type spécifique de seringue (1) au moyen du dispositif de saisie (150) et l'enregistrement de cette information au moyen du dispositif
d'enregistrement (140);
b) le déplacement d'une section du coulisseau, au moyen d'un mouvement du coulisseau (103) vers une position située entre la première position terminale (B) et la seconde position terminale (C), notamment vers une position située entre le premier capteur (111) et le second capteur (113), notamment à une première vitesse;
c) le déplacement de l'élément (105) au moyen du coulisseau (103) de manière à ce que le piston (5) atteigne, lorsque la seringue est insérée, la première position terminale (B), ou jusqu'à ce qu'une section prédéterminée du coulisseau (103), notamment un aimant permanent (115), ait atteint la première butée d'arrêt (A), notamment à une seconde vitesse;
où, lorsqu'une section prédéterminée du coulisseau (103) atteint ou dépasse la position du second capteur (113), le nombre de pas motorisés émis par le dispositif de comptage (120) pour ou à cette position est considéré comme une première valeur de comptage (ZW1),
et où, lorsque le piston (5) atteint la première position terminale (B) ou lorsque la partie du coulisseau (103) atteint la première butée d'arrêt (A), les pas motorisés, comptés au moyen du dispositif de comptage (120) jusqu'à ce que soit atteinte la position de la première position terminale (B) ou la première butée d'arrêt (A), sont considérés comme une seconde valeur de comptage (ZW2);
d) le calcul d'une valeur d'calibrage (K2) du second capteur (113) à partir de la première valeur de comptage (ZW1) et de la seconde valeur de comptage (ZW2) et l' enregistrement de cette valeur de calibrage (K2) pour le second capteur (113) au moyen du dispositif d'enregistrement (140);
e) éventuellement: la réinitialisation du dispositif de comptage, par exemple à "0" ou à une valeur de départ prédéterminée;
f) le déplacement de l'élément (105), notamment à la seconde ou à une troisième vitesse, en direction de la seconde position terminale (C);
g) la détermination du nombre de pas motorisés que le dispositif de comptage (120) délivre en tant que troisième valeur de comptage (ZW3), lorsqu'il atteint ou dépasse la position du premier capteur (111) au moyen de la section prédéterminée du coulisseau (103);
h) le calcul d'une valeur de calibrage (K1) du premier capteur (111) à partir de la troisième valeur de comptage (ZW3) et éventuellement d'au moins une autre valeur du groupe constitué de la première valeur de comptage (ZW1), de la seconde valeur de comptage (ZW2) ainsi que de la valeur de calibrage (K2) du second capteur (113), et l'enregistrement de cette valeur de calibrage (K1) pour le premier capteur (111) au moyen du dispositif d'enregistrement (140).

9. Le pousse-seringue médical (100) selon la revendication 8, où lors du déplacement selon l'étape b), la section du coulisseau (103) est d'abord déplacée en direction de la première butée d'arrêt (A), et la section du coulisseau (103) continue à se déplacer à la seconde vitesse après avoir atteint soit la position du second capteur (113), soit la première butée d'arrêt (A);
où, après avoir atteint la position du second capteur (113) ou après avoir atteint la première butée d'arrêt (A), la section du coulisseau (103) est automatiquement déplacée d'un nombre prédéterminé de pas motorisés en direction de la seconde butée d'arrêt (D) avant d'être ensuite déplacée selon l'étape c).

10. Le pousse-seringue médical (100) selon au moins l'une quelconque des revendications 8 à 9, où le nombre déterminé de pas motorisés est équilibré par une valeur de compensation et est ainsi compensé dans le calcul des valeurs de calibrage (K1, K2) du premier capteur (111) et/ou du second capteur (113) selon les étapes d) et g).

11. Le pousse-seringue médical (100) selon la revendication précédente, où les valeurs de calibrage (K1, K2) du premier capteur (111) et/ou du second capteur (113) ainsi compensées sont vérifiées quant à leur plausibilité.

12. Le pousse-seringue médical (100) selon au moins l'une quelconque des revendications 8 à 11, où le premier capteur (111) et/ou le second capteur (113) est/sont un capteur de champ magnétique, notamment un capteur à effet Hall, ou un capteur optoélectronique, notamment une barrière lumineuse.

13. Le pousse-seringue médical (100) selon au moins l'une quelconque des revendications 8 à 12, où le coulisseau (103) comprend un aimant permanent (115) déplaçable à une distance constante de la partie du coulisseau (103).

14. Le pousse-seringue médical (100) selon au moins l'une quelconque des revendications 8 à 13, où l'information concernant le type spécifique de seringue inclut au moins la distance (hₘₐₓ) entre la première position terminale (B) et la seconde position terminale (C) et/ou un facteur de correction et/ou un décalage.

15. Le pousse-seringue médical (100) selon au moins l'une quelconque des revendications 8 à 14, conçu comme un pousse-seringue à héparine.

16. Un appareil de traitement du sang, comprenant ou étant relié en communication de signal avec un pousse-seringue médical (100) selon l'une quelconque des revendications 8 à 15.

17. L'appareil de traitement du sang selon la revendication 16, conçu comme un appareil de dialyse péritonéale, un appareil d'hémodialyse, un appareil d'hémofiltration ou un appareil d'hémodiafiltration, notamment comme un appareil pour la thérapie de remplacement rénal chronique ou pour la thérapie de remplacement rénal continu (CRRT = continuous renal replacement therapy).

18. Un support d'enregistrement numérique, notamment sous la forme d'une disquette, d'un CD ou d'un DVD ou d'une EPROM comprenant des signaux de commande lisibles électroniquement, configuré pour interagir avec un système informatique programmable de manière à déclencher les étapes automatiques d'un procédé fondé sur l' invention selon l'une quelconque des revendications 1 à 7.

19. Un produit de programme informatique sous forme d'onde de signal ou ayant un code de programme enregistré sur un support lisible par une machine, pour déclencher les étapes automatiques du procédé fondé sur l'invention selon l'une quelconque des revendications 1 à 7, lorsque le produit de programme informatique s'exécute sur un ordinateur.
